# EUROPÄISCHE PATENTSCHRIFT

(11) **EP 0 951 261 B1**
(45) Veröffentlichungstag und Bekanntmachung des Hinweises auf die Patenterteilung: **15.05.2002**
(21) Anmeldenummer: 98902947.5
(22) Anmeldetag: 10.01.1998
(51) Int. Cl.: A61F 11/00, A61F 2/18

(54) **DRUCKAUSGLEICHVORRICHTUNG ALS PROTHETISCHER ERSATZ FÜR EINE EUSTACHISCHE RÖHRE**
PRESSURE-EQUALIZING DEVICE AS A PROSTHETIC REPLACEMENT FOR A EUSTACHIAN TUBE
DISPOSITIF D'EQUILIBRAGE DE PRESSION SERVANT DE PROTHESE D'UNE TROMPE D'EUSTACHE

(30) Priorität: 13.01.1997 DE 19700814
(43) Veröffentlichungstag der Anmeldung: 27.10.1999
(73) Patentinhaber: Stennert, Eberhard, 50935 Köln (DE)
(72) Erfinder: STENNERT, Eberhard, D-50935 Köln (DE); WALGER, Martin, D-50354 Hürth (DE); MEISTER, Hartmut, D-50739 Köln (DE)
(74) Vertreter: Bauer, Wulf, Dr.
(86) Internationale Anmeldenummer: DE9800072
(87) Internationale Veröffentlichungsnummer: WO9830183

(56) Entgegenhaltungen:
- WO-A-93/10729
- DE-A- 4 407 847
- US-A- 4 015 607
- US-A- 5 108 464

## Beschreibung

Die Erfindung bezieht sich auf eine Druckausgleichsvorrichtung, die als prothetischer Ersatz für eine Eustachische Röhre implantierbar ist.

Durch verschiedene Krankheiten oder Mißbildungen kann es dazu kommen, dass der Mitteiohrraum nicht ausreichend über die Eustachische Röhre belüftet wird. So ist bei Patienten mit chronischen Mittelohrentzündungen, chronischer Knocheneiterung, Cholesteatomen oder Mittelohr-Mißbildungen häufig die Notwendigkeit gegeben, die Eustachische Röhre durch eine entsprechende Prothese zu ersetzen. Diese muß den durch die natürliche Eustachische Röhre bewirkten Druckausgleich schaffen. Insbesondere ist eine derartige Druckausgleichsvorrichfung notwendig bei Mittelohrprothesen. Hierzu wird auf die Anmeldung vom gleichen Tag des selben Anmelders mit dem Titel "Mittelohrprothese" verwiesen.

Bekannt ist aus der WO-A-9310729 ein prothetischer Ersatz für die Eustachische Röhre. Darin ist ein Belüftungsrohr beschrieben, welches als Druckausgleichsvorrichtung für das Mittelohr vorgesehen ist. Das Belüftungsrohr umfaßt miteinander dicht verbundene Außen-, Innen- und Filterteile. Das Innenteil wird durch Verschraubung fest im Schädelknochen verankert. Das Außenteil wird durch Verschraubung mit dem Innenteil dicht verbunden. Das Filterteil kann auf das außenliegende Ende des Außenteils aufgesteckt werden. Die außenliegende Seite des Filterteils ist mit einem luftdurchlässigen, aber für Keime bzw. Wasser etc. undurchlässigen Filter versehen, das zur Außenwelt hin gerichtet angeordnet ist.

Dieses außenliegende Filterteil muß gleichzeitig das Mittelohr vor grober Verschmutzung durch Staub, Sand, Wasser etc. verhindern, übt also eine grobe Filterwirkung aus, um das Mittelohr vor Infektionen durch Keime zu schützen, insbesondere um gegen eindringendes Wasser und Keime schützen zu können, muß das Filterteil als extrem feinporiges Filter ausgeführt sein. Durch die außenliegende Anordnung des Filterteils ist das sehr feinporige Filter stark durch Verstopfung durch grobe Teilchen wie Schmutz oder Staub bedroht.

Aufgabe der vorliegenden Erfindung ist es, eine Druckausgleichsvorrichtung zu schaffen, die die natürliche Eustachische Röhre ersetzt, wenn diese nicht für die Belüftung des Mittelohrs zur Verfügung steht, wobei gleichzeitig die Lebensdauer verwendeter Filter erhöht und die Sterilität bei einem eventuellen Filterwechsel verbessert werden sollen.

Gelöst wird diese Aufgabe durch eine Druckausgleichsvorrichtung als prothetischer Ersatz für eine Eustachische Röhre, mit einem Außenteil und einem Innenteil, die miteinander über einen ersten Schlauch dicht verbunden sind, wobei das Außenteil 1. in einem Knochen verankerbar ist und einen gegenüber der umliegenden. Haut frei zugänglichen Bereich aufweist, 2. eine Aufnahme für einen Filter hat, die von außen zugänglich ist und 3. einen innenseitigen Anschluß für den ersten Schlauch aufweist und das Innenteil ein dichtes Gehäuse hat, das in zwei Kammern, nämlich eine Außenkammer und eine Innenkammer, unterteilt ist, die entweder durch ein Feinfilter oder durch eine Druckausgleichsmembran voneinander separiert sind und von denen die Außenkammer über einen Anschluß mit dem ersten Schlauch kommuniziert und die Innenkammer über einen Anschluß für einen zweiten Schlauch verbunden ist, der im Mittelohr endet.

Die erfindungsgemäße Druckausgleichsvorrichtung ermöglicht eine Belüftung der Paukenhöhle, also des Mittelohrs von einem von außen zugänglichen Teilbereichs des Kopfes her. Hierzu ist das Außenteil so in einen Knochen eingesetzt und seitlich von Haut umwachsen, dass es teilweise nach außen und damit von außen frei zugänglich hervorragt. Es wird hierfür eine ähnliche Technik verwand, wie sie für knochenimplantierte Hörgeräte bereits bekannt ist. Vorzugsweise ist das Außenteil hinter dem Ohr und durch die Ohrmuschel möglichst abgedeckt angeordnet.

Das Außenteil dient als Lufteinlaß, es ist weiterhin dafür zuständig, groben Schmutz und Wasser von den weiteren Teilen der Druckausgleichsvorrichtung fern zu halten. Hierzu hat es einen Filter, der in einer Aufnahme des Außenteils untergebracht ist und vorzugsweise auswechselbar ist.

Das Außenteil ist über den ersten Schlauch mit dem Innenteil verbunden. Letzteres hat die Aufgabe, eine keimdichte Trennung zu bewirken. Das Innenteil hat ein Gehäuse, das in zwei Kammern unterteilt ist. Diese sind entweder durch eine hochflexible, aber völlig undurchlässige Membran voneinander separiert oder über ein Feinfilter miteinander verbunden. Die beiden Kammern des Gehäuses werden als Außenkammer und als Innenkammer bezeichnet, die Außenkammer ist über den ersten Schlauch mit dem Außenteil verbunden. Die Innenkammer ist über den zweiten Schlauch mit dem Mittelohr verbunden, vorzugsweise mit einer dort vorgesehenen Mittelohrprothese.

Durch die hochflexible Membran wird sichergestellt, dass von der Außenkammer keine Keime in die Innenkammer gelangen können. Die gleiche Aufgabe hat das Feinfilter, das aber zudem noch luftdurchlässig ist. Um dem Feinfilter eine möglichst große Filterfläche geben zu können, ist es vorzugsweise als Hohlfaserfilter ausgeführt. Zu Hohlfaserfiltern allgemein wird auf die US-Patentschriften 5 108 464 und 5 002 590 und insbesondere die dort genannte Literatur verwiesen.

Weitere Vorteile und Merkmale der Erfindung ergeben sich aus den übrigen Patentansprüchen sowie der nun folgenden Beschreibung von nicht einschränkend zu verstehenden Ausführungsbeispielen, die unter Bezugnahme auf die Zeichnung näher erläutert werden. In dieser zeigen:
- FIG. 1:: eine schnittbildliche Seitenansicht einer Mittelohrprothese mit der erfindungsgemäßen Druckausgleichsvorrichtung, diese ist mit einer hochflexiblen Membran versehen,
- FIG. 2:: eine Darstellung der Druckausgleichsvorrichtung entsprechend Figur 1, jedoch nunmehr mit einem Feinfilter im Innenteil und
- FIG. 3:: eine schnittbildliche Darstellung eines Innenteils, das mit Hohlfasern als Feinfilter ausgerüstet ist.

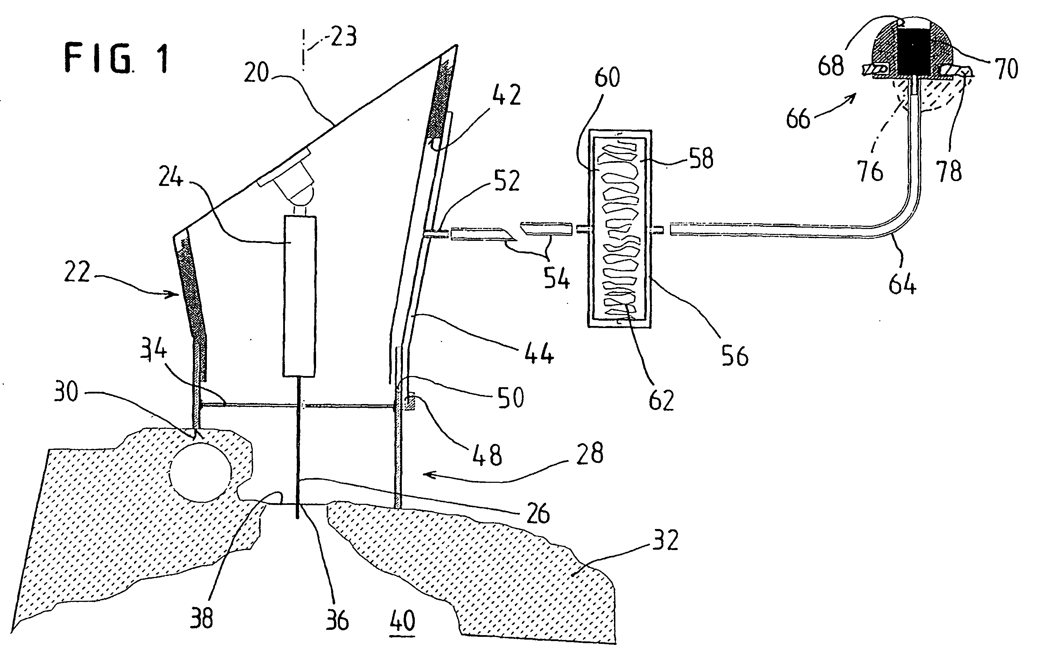

In Figur 1 ist eine Druckausgleichsvorrichtung zusammen mit einer Mittelohrprothese dargestellt. Beide werden nun im folgenden beschrieben:

Die Mittelohrprothese hat ein künstliches Trommelfell 20, das im wesentlichen einen ovalen Zuschnitt hat. Der Fläche nach ist es größer als die Fläche eines normalen menschlichen Trommelfells, beispielsweise 1,5 mal so groß. Es ist aus einem für den Einsatz als Prothese biologisch geeigneten Material zugeschnitten.

Das Trommelfell 20 ist dicht verbunden mit einem zweiteilig ausgebildeten Gehäuse, konkret ist es mit einem Übertragungsteil 22 verbunden. Dieses ist im wesentlichen rohrförmig. Es hat einen im wesentlichen zylindrischen Bereich, der in Figur 1 unten dargestellt ist und einen sich von diesem aus erweiternden Bereich, der im wesentlichen auf einem Kegelmantel verläuft. Der Durchmesser des zylindrischen Bereichs liegt bei etwa 6 mm, der sich erweiternde Bereich hat entsprechende Abmessungen von etwa 11 mm. Wie Figur 1 zeigt, befindet sich das Trommelfell 20 in einer Ebene, sie verläuft in einem Winkel 37° zu einer Längsachse 23 des Übertragungsteils 22. Durch die Schrägstellung des Trommelfells 20 hat das Trommelfell 20 eine gegenüber dem normalen Ohr größere Fläche.

Auf der Innenseite des Trommelfells 20, vorzugsweise im Flächenmittelpunkt, ist ein zweites Ossikelteil 24 dauerhaft befestigt. Im konkreten Ausführungsbeispiel wird ein handelsüblicher Ossikelersatz der Firma Richards GmbH eingesetzt. Er hat in Nähe des Trommelfells 20 ein Kugelgelenk, darunter befindet sich ein Hohlschaft für die Aufnahme eines noch zu besprechenden ersten Ossikelteils. Das zweite Ossikelteil 24 verläuft unterhalb des Kugelgelenkes zentrisch zur Mittelachse des Übertragungsteils 22.

Weiterhin gehört zum Gehäuse ein Ankopplungsteil 28. Es ist insgesamt etwas kleiner als das Übertragungsteil 22 und mit diesem gemeinsam so ausgebildet, das beide Teile einfach, dicht und dauerhaft miteinander verbunden werden können, insbesondere durch Stecken in Längsrichtung und Kleben. Das Ankopplungsteil 28 ist im wesentlichen zylindrisch, sein Durchmesser liegt ebenfalls bei etwa 6 mm. Es hat einen freien Endbereich 30, der einen Verlauf hat, welcher dem jeweiligen individuellen Relief der Paukenwand eines Träger der Mittelohrprothese möglichst genau nachgebildet ist. Wie bereits oben ausgeführt wurde, gibt es mehrere Verfahren, um den Verlauf des individuellen Reliefs der Paukenwand zu ermitteln bzw. abzutasten. Hierzu wird auch auf die Patentanmeldung "Vorrichtung zum mechanischen Abtasten und Erfassen des Verlaufs der Paukenwand eines Mittelohrs" des selben Anmelders und vom selben Anmeldetag verwiesen, der Offenbarungsgehalt dieser Anmeldung wird zum Gegenstand der Offenbarung der vorliegenden Anmeldung gemacht.

Der freie Endbereich 30 ist demgemäß so ausgeführt, dass er präzise an der Paukenwand 32 anliegt, wie dies aus Figur 1 ersichtlich ist. Auf diese Weise ist ein hermetisch dichter Abschluß an dieser Stelle möglich, es kann dünnflüssiges Befestigungs- und Dichtmaterial verwendet werden, um die Verbindung zwischen dem freien Endbereich 30 und der Paukenwand 32 zu realisieren.

Im Ankopplungsteil 28 ist eine Halteeinrichtung 34 für das erste Ossikelteil 26 vorgesehen. Letzteres ist als ein dünner Golddraht ausgeführt, der durch eine sehr kleine, künstlich geschaffene Öffnung 36 in einer Stapes-Fußplatte 38 gerade passend hindurch reicht und somit mit seinem unteren, freien Endbereich in das Innenohr 40 hineinragt. Im gezeigten Ausführungsbeispiel ist die Halteeinrichtung 34 ein im wesentlichen diagonal verlaufender Draht, an dem das erste Ossikelteil 26 festliegt bzw. festlegbar ist. Während der Implantation der Mittelohrprothese wird die Halteeinrichtung 34 durchtrennt, so dass sich das mit dem zweiten Ossikelteil 24 verbundene erste Ossikelteil 26 durch sie frei hin- und her bewegen kann.

Bei dem hier gezeigten Ausführungsbeispiel der Mittelohrprothese ist für die Verbindung der beiden Ossikelteile 24, 26 und auch für das Durchtrennen der Halteeinrichtung 34 ein Zugang in den Innenraum notwendig. Andere Konstruktionen ohne einen derartigen Zugang sind möglich. Für diesen Zugang ist im Übertragungsteil 22 ein Fenster 42 vorgesehen, das durch eine Abdeckplatte 44 verschließbar ist.

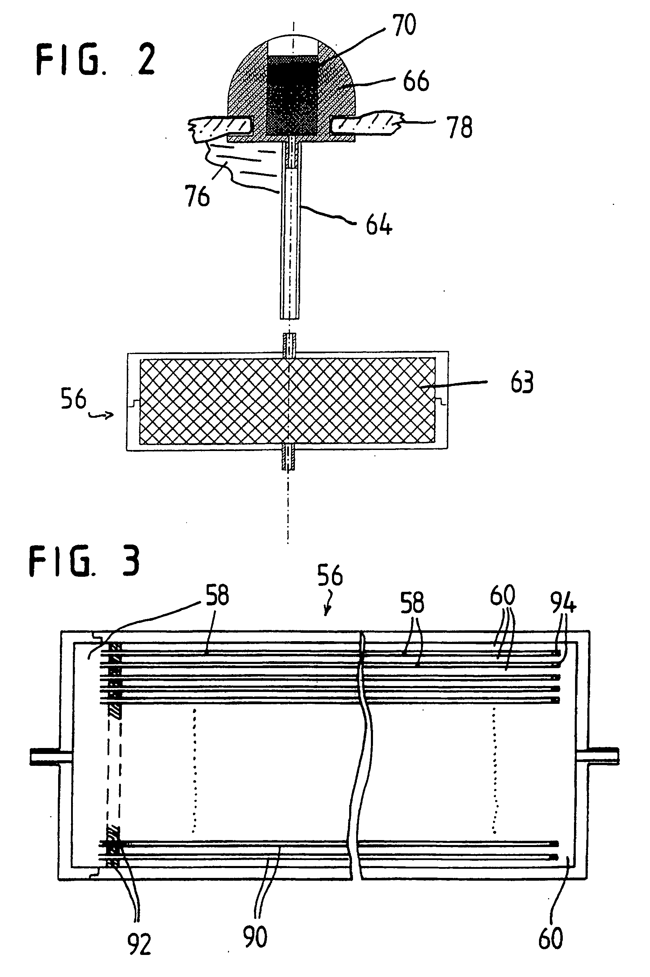

Wie Figur 1 zeigt, hat die Abdeckplatte 44 einen Anschluß 52. Er ist für einen Schlauch 54 vorgesehen. Der Schlauch 54 führt zu einem Innenteil 56 einer Druckausgleichsvorrichtung. Dieses Innenteil 56 ist als ein dosenförmiges Gehäuse ausgeführt, es hat zwei Kammern, nämlich eine Außenkammer 58 und eine Innenkammer 60. Beide sind durch eine sehr flexible Membran 62 hermetisch voneinander getrennt. In einer anderen Ausführung sind sie durch ein Feinfilter 63, das auch gegen Bakterien und Mikroben dicht ist, aber luftdurchlässig ist, voneinander separiert. Als Feinfilter 63 kommen hier insbesondere Hohlfaserfilter in Frage, siehe Fig. 3.

Über einen weiteren Schlauch 64 ist die Außenkammer 58 mit einem Außenteil 66 verbunden. Es ist in einem Knochen 76 verankert und teilweise von außen zugänglich, siehe Haut 78. Es wird hier eine Konstruktion verwand, wie sie ähnlich ist bei sogenannten knochenleitenden Hörgeräten. Das Außenteil 66 hat eine Ausnehmung 68, in die ein Filter 70 eingesetzt ist. Dieses ist vorzugsweise austauschbar. Das Filter 70 verhindert, dass Wasser, grober Schmutz usw. in den Schlauch 64 gelangen kann. Die Barriere gegen Keime und dergleichen wird durch die Membran 62 bzw. das an ihre Stelle tretende Feinfilter erreicht.

Im Ausführungsbeispiel nach den Figuren 2 und 3 ist das Innenteil nicht mit einer hochflexiblen Membran 62 versehen, vielmehr sind die beiden Kammern 58, 60 nunmehr durch ein Feinfilter 63 voneinander getrennt. Dieses ist so ausgeführt, dass keinerlei Keime von der Außenkammer 58 in die Innenkammer 60 gelangen können.

Da das Innenteil nach dem Einsetzen der erfindungsgemäßen Prothese nicht mehr unmittelbar zugänglich ist, vielmehr nur durch einen erneuten operativen Eingriff zugänglich wird, ist sehr großen Wert auf die mechanische Ausführung gelegt. So sind die Verbindungen zwischen den Schläuchen 54, 64 und den zugehörigen Anschlüssen dauerhaft ausgeführt. Das Gehäuse des Innenteils ist dauerhaft hermetisch verschlossen. Die Membran 62 hat eine sehr hohe Beständigkeit. Gleiches gilt für das Feinfilter 63.

Figur 3 zeigt ein Innenteil, in dessen Gehäuse eine große Anzahl von Hohlfasern 90 angeordnet sind. Sie bilden gemeinsam das Feinfilter. Sie verlaufen im wesentlichen parallel zueinander, sie können dicht an dicht aneinanderliegen, da schon aufgrund ihrer kreisrunden Form zwischen den einzelnen Hohlfasern Freiräume verbleiben, die in der gezeigten Darstellung zur Innenkammer 60 gehören. Die Innenkammer kann aber auch alternativ mit den Innenräumen der Hohlfasern 90 in Verbindung stehen. In der Zeichnung gemäß Figur 3 sind die Abstände zwischen den einzelnen Hohlfasern groß gezeichnet, um die Zeichnung besser lesen zu können. Wenn sich die Hohlfasern 90 nicht berühren sollen, so ist der Abstand zwischen ihnen möglichst gering gehalten.

Wie Figur 3 zeigt, sind die Hohlfasern 90 insgesamt am linken Endbereich so von einem Stopfen 92 umgossen, dass der Zugang zu ihren Innenräumen von links gesehen frei bleibt. Der Stopfen 92 ummantelt jede einzelne Hohlfaser dicht im Bereich ihres Außenmantels und schließt dicht gegenüber der Innenwand des Gehäuses ab.

Am rechten Endbereich ist jede einzelne Hohlfaser 90 verschlossen, beispielsweise durch Eintauchen in einen entsprechenden, flüssigen Kunststoff oder durch Zusammenquetschen. In der Figur ist dieser Abschluß durch einzelne Pfropfen 94 dargestellt. Sie stellen sicher, dass jede einzelne Hohlfaser an ihrem rechten Endbereich (in der Figur 3) hermetisch verschlossen ist.

Hohlfasermembranen der hier in Rede stehenden Art sind beispielsweise aus dem US-Patent 4,781,733 einschließlich des auf der Frontseite dieser Patentschrift aufgelisteten Standes der Technik bekannt.

## Patentansprüche

1. Druckausgleichsvorrichtung als prothetischer Ersatz für eine Eustachische Röhre, mit einem Außenteil (66) und einem Innenteil (56), die miteinander über einen ersten Schlauch (64) dicht verbunden sind, wobei das Außenteil (66) erstens in einem Knochen (76) verankerbar ist und einen gegenüber der umliegenden Haut (78) frei zugänglichen Bereich aufweist, zweitens eine Aufnahme für einen Filter hat, die von außen zugänglich ist und drittens einen innenseitigen Anschluß (52) für den ersten Schlauch (64) aufweist und wobei das Innenteil (56) ein dichtes Gehäuse hat, das in zwei Kammern, nämlich eine Außenkammer (58) und eine Innenkammer (60), unterteilt ist, die entweder durch ein Feinfilter (63) oder durch eine Druckausgleichsmembran voneinander separiert sind und von denen die Außenkammer (58) über einen Anschluß (52) mit dem ersten Schlauch (64) kommuniziert und die Innenkammer (60) über einen Anschluß (52) für einen zweiten Schlauch (54) verbunden ist, der im Mittelohr endet.

2. Druckausgleichsvorrichtung nach Anspruch 1, **dadurch gekennzeichnet, dass** das Filter austauschbar in der Aufnahme untergebracht ist.

3. Druckausgleichsvorrichtung nach Anspruch 1, **dadurch gekennzeichnet, dass** das Feinfilter (63) bakterien- und virendicht, aber luftdurchlässig ist, insbesondere, dass das Feinfilter (63) als ein Hohlfaserfilter ausgeführt ist.

4. Druckausgleichsvorrichtung nach Anspruch 1, **dadurch gekennzeichnet, dass** die Schläuche als Silikonschläuche ausgeführt sind.

5. Druckausgleichsvorrichtung nach Anspruch 1, **dadurch gekennzeichnet, dass** die Druckausgleichsmembran eine dünne, dichte und hochflexible Folie ist.

6. Druckausgleichsvorrichtung nach einem der Ansprüche 1 bis 5, **dadurch gekennzeichnet, dass** das Gehäuse im wesentlichen dosenförmig ist.

7. Druckausgleichsvorrichtung nach Anspruch 1, **dadurch gekennzeichnet, dass** das Innenteil (56) eine Abmessung und eine Ausbildung hat, die es für die Unterbringung im Mittelohr geeignet machen.

8. Druckausgleichsvorrichtung nach Anspruch 1, **dadurch gekennzeichnet, dass** die Innenkammer (60) ein Feinfilter (63) aufweist und dieses durch eine Vielzahl von Hohlfasern (90) realisiert ist, wobei die Hohlfasern (90) im wesentlichen parallel zueinander verlaufen, im wesentlichen gleiche Länge haben, an einem Endbereich jeweils einen Verschluß, insbesondere einen Pfropfen (94) haben, der sie individuell verschließt und die in der Nähe des anderen Endbereichs gemeinsam dicht von einem Stopfen (92) umschlossen sind, vorzugsweise eingegossen sind.

## Claims

1. Pressure compensation device as a prosthetic substitute for a eustachian tube with an outer part (66) and an inner part (56) that are tightly connected via a first tube (64), whereas the outer part (66) in the first place may be anchored in a bone (76) and has a freely accessible area compared with the surrounding skin (78), in the second place has a seat for a filter being accessible from the outside and in the third place is provided with an inside connection (52) for the first tube (64) and whereas the inner part (56) has a sealed housing divided into two chambers, namely into an outer chamber (58) and an inner chamber (60) separated from each other either by a fine filter (63) or by a pressure compensation membrane, the outer chamber (58) communicating with the first tube (64) via a connection (52) and the inner chamber (60) being connected via a connection (52) with a second tube (54) ending in the middle ear.

2. Pressure compensation device according to claim 1, **characterized in that** the filter is arranged in the seat and is exchangeable.

3. Pressure compensation device according to claim 1, **characterized in that** the fine filter (63) is sealed against bacteria and virus but permeable to air and particularly that the fine filter (63) is designed as a hollow fibre filter.

4. Pressure compensation device according to claim 1, **characterized in that** the tubes are designed as silicone tubes.

5. Pressure compensation device according to claim 1, **characterized in that** the pressure compensation membrane is a thin, tight and highly flexible foil.

6. Pressure compensation device according to one of the claims 1 to 5, **characterized in that** the housing is essentially boxlike.

7. Pressure compensation device according to claim 1, **characterized in that** the inner part (56) has a size and a design appropriate to its location in the middle ear.

8. Pressure compensation device according to claim 1, **characterized in that** the inner chamber (60) has a fine filter (63) made of a plurality of hollow fibres (90), whereas the hollow fibres (90) are essentially running parallel to one another, have essentially the same length, have each on one end side a closure, particularly a stopper (94) that closes each of the fibres and whereas all the fibres are tightly encompassed, preferably by casting, in the vicinity of their other end by a plug (92).

## Revendications

1. Dispositif de compensation de pression servant de prothèse d'une trompe d'Eustache, comprenant une partie extérieure (66) et une partie intérieure (56) qui sont reliées l'une à l'autre de manière étanche par un premier tuyau flexible (64), la partie extérieure (66) pouvant, premièrement, être ancrée dans un os (76) et présentant une zone librement accessible par rapport à la peau environnante (78), ayant deuxièmement un logement pour un filtre, qui est accessible de l'extérieur, et présentant troisièmement un raccord (52) situé du côté intérieur pour le premier tuyau flexible (64), et la partie intérieure (56) présentant un boîtier étanche qui est divisé en deux chambres, à savoir une chambre extérieure (58) et une chambre intérieure (60) qui sont séparées l'une de l'autre soit par un filtre fin (63) soit par une membrane de compensation de pression et dont la chambre extérieure (58) communique par un raccord (52) avec le premier tuyau flexible (64) et la chambre intérieure (60) est reliée par un raccord (52) pour un deuxième tuyau flexible (54) qui se termine dans l'oreille moyenne.

2. Dispositif de compensation de pression selon la revendication 1, **caractérisé par le fait que** le filtre est placé dans le logement de manière à pouvoir être échangé.

3. Dispositif de compensation de pression selon la revendication 1, **caractérisé par le fait que** le filtre fin (63) est étanche aux bactéries et aux virus, mais perméable à l'air, en particulier que le filtre fin (63) est réalisé comme un filtre de fibres creuses.

4. Dispositif de compensation de pression selon la revendication 1, **caractérisé par le fait que** les tuyaux flexibles sont réalisés en tant que tuyaux flexibles en silicone.

5. Dispositif de compensation de pression selon la revendication 1, **caractérisé par le fait que** la membrane de compensation de pression est une feuille mince, étanche et hautement flexible.

6. Dispositif de compensation de pression selon l'une des revendications 1 à 5, **caractérisé par le fait que** le boîtier présente pour l'essentiel la forme d'une boîte.

7. Dispositif de compensation de pression selon la revendication 1, **caractérisé par le fait que** la partie intérieure (56) est dimensionnée et réalisée de telle manière qu'elle se prête à être logée dans l'oreille moyenne.

8. Dispositif de compensation de pression selon la revendication 1, **caractérisé par le fait que** la chambre intérieure (60) présente un filtre fin (63) et que celui-ci est réalisé par une multiplicité de fibres creuses (90), lesdites fibres creuses (90) s'étendant pour l'essentiel parallèlement l'une à l'autre, présentant pour l'essentiel la même longueur, présentant sur une zone d'extrémité respectivement une fermeture, en particulier un tampon (94) qui les ferme individuellement, et, à proximité de l'autre zone d'extrémité, les fibres étant entourées ensemble, de préférence par moulage, de manière étanche par un bouchon (92).
